# EUROPEAN PATENT SPECIFICATION

(11) **EP 2 628 487 B1**
(45) Date of publication and mention of the grant of the patent: **03.01.2018**
(21) Application number: 13154937.0
(22) Date of filing: 12.02.2013
(51) Int. Cl.: A61K 45/06, A61P 17/00

(54) **Adjuvant composition for topical use**
Adjuvanszuzammensetzung zur topischen Verwendung
Composition d'adjuvant à usage topique

(30) Priority: 15.02.2012 IT MO20120036
(43) Date of publication of application: 21.08.2013
(73) Proprietor: Drex Pharma S.R.L., 20125 Milano (IT)
(72) Inventor: Delsignore, Giorgio, 13100 Vercelli (IT); Masci, Silvio, 65100 Pescara (IT); Cammisa, Anna, Milano (IT); Bruschi, Pietro, 20122 Milano (IT)
(74) Representative: Crugnola, Pietro

(56) References cited:
- WO-A1-03/084553
- WO-A1-2005/058273
- WO-A2-2010/044076
- WO-A2-2012/112796
- IT-A1- MI20 070 721

## Description

The present invention relates to an adjuvant composition for topical use, in particular an adjuvant composition having soothing and anti-inflammatory properties, which is usable for treating skin disreactivity in which inflammatory phenomena occur.

"Skin disreactivity" is intended as an abnormal - inflammatory or immune - response of the skin tissues to an exogenous or endogenous stimulus.

In medicine, the term "dermatitis" is generically attributed to a plurality of acute or chronic inflammatory processes of the skin. In general, a skin inflammation manifests itself as an irritative state of the skin and is accompanied by symptoms comprising:
- reddening of the skin, located in specific areas and accompanied by pain, itching and possible blisters that enclose liquids;
- light red blotches, having variable shape and size and at which the skin is scaly. In some cases, these skin eruptions have a chronic trend, alternating periods of remission and recrudescence;
- peeling of the skin in the hairy areas, with production of dry or greasy scales.

The causes, which can produce a skin inflammation, are many and of different nature and they may comprise:
- allergies caused by a hyperactivity of the cells constituting the immune system (so-called contact dermatitis);
- autoimmune reactions (which would intervene, for example, in the case of psoriasis);
- chemical agents;
- physical agents (exposure to sun, wind, thermal fluctuations) .

The skin inflammatory process usually develops and evolves according to a preset sequence, comprising the following steps:
- initial alteration of the tissue (tissular damage);
- vasodilation and increase of the permeability of the blood vessels;
- exit of plasma and blood cells through the vessel wall;
- cellular proliferation (macrophages, leucocytes, local cells, etc.);
- tissular repair (or regeneration).

Therefore, the skin inflammatory process can be considered as a response that the skin defence system produces for opposing the received - microbial, chemical, physical, (auto) immune - insult. The aforesaid response is directed to obtain a localisation (namely, a reduction of extension) of the irritative phenomenon, a dilution (namely, a reduction of intensity) of the irritative phenomenon, an elimination of the damaged tissue, as well as a repair and a functional recovery of the damaged tissue.

In the inflammatory processes, many chemical mediators are involved that have different functions and are able to trigger various defence mechanisms.

A list of the aforesaid chemical mediators, and of the respective functions, is set out in Table A below:

**Table A**

| *Function* | *Mediators* |
|---|---|
| Increase of the vascular permeability of the small blood vessels | histamine,serotonin,bradykinin, C3a, C5a, PGE₂, LTC₄, LTD₄, prostacyclin, activated Hagerman factor, high-molecular-weight kininogen fragments, fibrinopeptides |
| Vasoconstriction | TXA₂, LTB₄, LTC₄, LTD₄, C5a, N-formyl peptides |
| Smooth muscle contraction | C3a, C5a, histamine, LTB₄, LTC₄, LTD₄, TXA₂, serotonin, PAF, bradykinin |
| Increase of the adhesiveness of the endothelial cells | IL-1, TNF-α, MCP, endotoxins, LTB₄ |
| Degranulation of mastocytes | C5a, C3a |
| *In phagocytes*: | |
| Proliferation of stem cells | IL-3, G-CSF, GM-CSF, M-CSF |
| Recruitment from bone marrow | CSF₈, IL-1 |
| Adhesion/aggregation | iC3b, IgG, fibronectin, lectins |
| Chemotaxis | C5a, LTB₄, IL-8 and other chemokines, PAF, histamine (for eosinophil granulocytes), N-formyl peptides, laminin, collagen fragments, lymphocyte-derived chemotactic factor, fibrinopeptides |
| Release of lysosomal granules | C5a, IL-8, PAF, most chemoattractants, phagocytosis |
| Production of reactive oxygen intermediates | C5a, TNF-α, PAF, IL-8, phagocyte particles; production is increased by IFN-γ |
| Phagocytosis | C3b, iC3b, IgG (Fc portion), fibronectin; the expression of the Fc receptor is increased by IFN-γ |
| Formation of granulomes | IFN-γ, TNF-α, IL-1 |
| Pyrogens | IL-1, TNF-α, PGE₂, IL-6 |
| Pain | PGE₂, bradykinin |

From what has been disclosed above, it is clear that the inflammatory phenomena that may affect the skin are extremely relevant, both for the size and extension of the symptoms and for the consequent substantial alteration of the quality of life of the patients affected by these skin pathologies.

For the medical treatment of the dermatitises, pharmaceutical compositions for topical use have been developed and are still used. These compositions are based on anti-inflammatory agents such as, for example, corticosteroids.

A drawback connected to the topical use of corticosteroids consists of the fact that the patients treated with these drugs are subject to undesired, and not negligible, side effects such as, for example: cutaneous atrophy, hypertrichosis, steroid acne, telangiectasias, sensitization phenomena.

In addition to the above-mentioned pharmaceutical compositions, also non-pharmaceutical compositions have been made and commercially proposed - in particular, cosmetic compositions - which have a soothing effect and are usable as adjuvant compositions in the treatment of dermatitises.

In the pharmaceutical and cosmetic field 18-β-glycyrrhetic acid (glycyrrhetinic acid) is known, which has molecular formula C₃₀H₄₆O₄ and a structure formula set below:

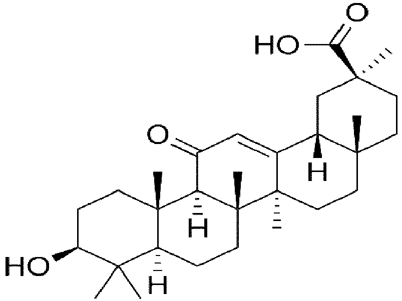

18-β-glycyrrhetic acid is a pentacyclic triterpenoid acid, which is obtainable by hydrolysis of the glycyrrhizic acid (or glycyrrhizin), which in turn is a glycoside extracted from the root of *Glycyrrhiza glabra* L. (liquorice).

18-β-glycyrrhetic acid is provided with anti-inflammatory activity, which is exploited - for example - to make antiacid compositions that are administrable *per os* and are usable in the symptomatic treatment of gastritis.

Soothing adjuvant compositions for topical use have also been proposed that incorporate 18-β-glycyrrhetic acid as anti-inflammatory active principle.

In comparison with the corticosteroid-based pharmaceutical compositions, known soothing compositions containing 18-β-glycyrrhetic acid do not produce the undesired side effects disclosed previously.

It has been nevertheless found that, in known soothing compositions containing 18-β-glycyrrhetic acid, the latter acts as anti-inflammatory active principle in a scarcely effective manner.

Therefore, known soothing adjuvant compositions containing 18-β-glycyrrhetic acid are unable to replace, or at least support effectively, corticosteroids in the treatment of dermatitises.

WO03/084553 A1 discloses pharmaceutical compositions for topical administration, which comprise proanthocyanidins combined with glycyrrhetinic acid and are usable for the treatment of mucosae inflammatory conditions.

ITMI20070721 A1 discloses compositions for treating dermatitises, comprising for example karite butter and stearyl glycyrrhetinate as soothing a gents.

WO2005/058273 A1 discloses the use of methylthioadenosine (MTA) for the preparation of compositions for the treatment of irritated, inflamed and/or erythematous skin. The compositions may comprise soothing and restorative agents, such as for example shea butter and potassium glycyrrhizinate.

WO2010/044076 A2 discloses cosmetic and topical compositions, comprising xanthohumol as active ingredient for brightening complexion and/or reducing cutaneous redness. The composition may comprise additional agents, such as for example dipotassium glycyrrhizinate and stearyl glycyrrhetinate.

WO2012/112796 A2 discloses a perfluorocarbon composition, which may comprise dipotassium glycyrrhizate and is usable for the topical treatment of pruritus.

An object of the invention is to improve known soothing adjuvant compositions.

Another object is to improve the soothing adjuvant compositions that are usable in the topical treatment of skin disreactivity, in particular skin disreactivity accompanied by inflammation.

A further object is to make available a soothing adjuvant composition that is provided with an effective anti-inflammatory activity.

According to the invention, a composition is provided for topical use, as defined in claim 1.

The composition according to the invention, if applied on a skin tissue affected by dermatitis, enables a new and unexpected synergic effect to be obtained, which was discovered during the course of clinical experimentations conducted on behalf of Drex Pharma S.r.l.

It has in fact been discovered that a composition comprising a hydrosoluble salt of the glycyrrhizic acid, a liposoluble ester of the 18-β-glycyrrhetic acid and a *Butyrospermum parkii* seed extract is able to produce a plurality of positive effects on the skin tissue, i.e. a soothing effect, an anti-inflammatory effect and an effect of tissular regeneration.

In particular, it has been verified that the aforesaid effects are quantitatively and qualitatively greater than those that can be reasonably expected on the basis of the presence, in the composition, of an anti-inflammatory agent (18-β-glycyrrhetic acid) and of an emollient, soothing and hydrating substance (*Butyrospermum parkii* seed extract).

The clinical experimentation has above all shown that, by treating the skin with the composition according to the invention, it is possible to achieve an anti-inflammatory effect that is comparable to that which is obtainable through the use of corticosteroids, without however causing the undesired side effects that are typical of these drugs.

The composition according to the invention can therefore be effectively used as a soothing and anti-inflammatory adjuvant composition in the treatment of skin disreactivity accompanied by eczemas and inflammations.

The aforesaid skin disreactivity comprises: allergic dermatitises, contact dermatitises, irritative dermatitises, atopic dermatitises, seborrhoeic dermatitises, radiodermatitises, solar erythemas, skin stresses (caused, for example, by beauty treatments such as depilation or peeling).

More in general, the composition according to the invention can be effectively used as an adjuvant composition in the treatment of skin pathologies accompanied by the release into the tissues of the main mediators of the inflammation.

The adjuvant composition according to the invention can be made, for example, in the form of a cream, fluid, emulsion, gel, gel-cream, serum-gel, paste, mousse and spray.

It is further possible to make a composition of cosmetic type - for example a nutrient or moisturising cream - incorporating the adjuvant composition according to the invention and thus also applicable to a skin affected by one of the above-mentioned skin disreactivity.

The chemical nature and the function of the active principles contained in the composition according to the invention are disclosed below.

The hydrosoluble salt of the glycyrrhizic acid is represented by dipotassium glycyrrhizate, having molecular formula C₄₂H₆₂O₁₆·2K and a structure formula set out below:

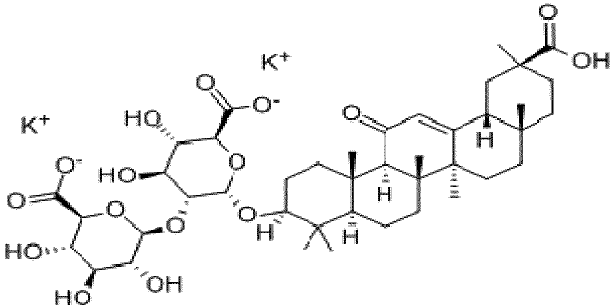

In the formula of the composition according to the invention, the hydrosoluble salt of the glycyrrhizic acid is present in percentage concentrations by weight varying from 0.1% to 10% and in particular comprised between 0.5% and 2%. In addition to dipotassium glycyrrhizate, it is possible to use disodium glycyrrhizate or ammonium glycyrrhizate as hydrosoluble salt of glycyrrhizic acid. The liposoluble ester of the 18-β-glycyrrhetic acid is represented by stearyl glycyrrhetinate, i.e. the stearic ester of the 18-β-glycyrrhetic acid. The stearyl glycyrrhetinate has molecular formula C₄₈H₈₂O₄ and a structure formula set out below:

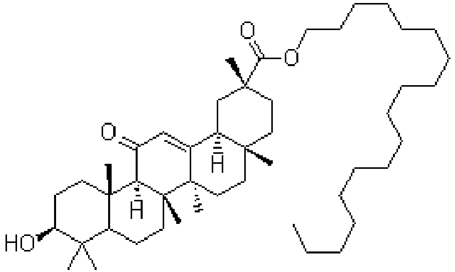

In the formula of the composition according to the invention, the liposoluble ester of the 18-β-glycyrrhetic acid is present in percentage concentrations by weight varying from 0.1% to 10% and in particular comprised between 0.2% and 2%.

In addition to the above-mentioned stearyl glycyrrhetinate, it is possible to use the ester of the glyceric acid, the ester of the succinic acid or the ester of the pyridoxine (vitamin B6) as liposoluble ester of the 18-β-glycyrrhetic acid. *Butyrospermum parkii* is a plant belonging to the family of the *Sapotaceae*, which is also classified as *Vitellaria paradoxa* and *Butyrospermum paradoxa*, from the seeds of which an extract known as "karité butter", "shea butter", or "galam butter" is obtained. Below, both in the description and in the claims, the terms "*Butyrospermum parkii* seed extract", *"Vitellaria paradoxa* seed extract", *"Butyrospermum paradoxa* seed extract", "karité butter", "shea butter" and "galam butter" are to be considered to be synonyms.

Shea butter is a vegetable extract that is rich in fatty acids (oleic acid, stearic acid, palmitoleic acid, linoleic acid) and triterpene esters, the use of which in the cosmetic field as an emollient, soothant and moisturiser is known. In the formula of the composition according to the invention, the shea butter is present in percentage concentrations by weight varying from 0.1% to 10% and in particular comprised between 0.25% and 2%.

In order to make the composition according to the invention, a type of shea butter has been used that is particularly enriched - up to 50% - in the triterpene fraction, i.e. enriched in triterpene esters. The latter derive from the esterificaton of fatty acids, acetic acid and cinnamic acid with triterpene alcohols (α-amyrin, β-amyrin, butyrospermol, parkeol, lupeol).

The aforesaid triterpene fraction is able to influence various physiological and pathological processes in the skin tissue. In particular, the triterpene fraction that is present in the composition according to the invention is able to increase the thickness of the epidermis (thus increasing the so-called barrier function thereof), to increase the collagen content of the dermis, to stimulate the proliferation of the fibroblasts, to inhibit the activity of protease enzymes produced during inflammation (collagenase and metalloproteinase), and to inhibit the release of the proinflammatory cytokines IL-1α.

The hydrosoluble salt of the glycyrrhizic acid, the liposoluble ester of the 18-β-glycyrrhetic acid and the *Butyrospermum parkii* seed extract are the essential active principles of the composition according to the invention.

In addition to the aforementioned active principles, the composition according to the invention can comprise a further active principle, namely hydrogenated lecithin, which is a phospholipid provided with a high emulsifying activity. The hydrogenated lecithin is able to act as a phospholipidic transporting and releasing system, because it is able to produce a phospholipidic environment that is suitable for promoting the transport of the hydrosoluble potassium salt and of the liposoluble ester of the 18-β-glycyrrhetic acid through the epidermis. In the formula of the composition according to the invention, the hydrogenated lecithin is present in percentage concentrations by weight varying from 0.1% to 10% and in particular comprised between 0.3% and 2%.

Owing to the properties of the active principles and to the combination thereof in the composition according to the invention, the latter is able to perform an anti-inflammatory activity that is substantially comparable with that performed by the corticosteroids, but without inducing the undesired side effects caused by these drugs.

In the Examples 1 to 6, set out below, some of the possible formulations of the composition according to the invention are disclosed. For each formulation, the percentage composition is shown and a corresponding preparation procedure is disclosed concisely.

In the Examples 1-6, each component is identified through the corresponding INCI (*International Nomenclature of Cosmetic Ingredients*) name. In the Tables of the Examples 1-6, in addition to the INCI names also corresponding chemical names or synonyms are indicated (in brackets).

### Example 1 - Adjuvant cream for topical use

In the Table 1 there is indicated the quali-quantitative composition corresponding to 100 grams of an adjuvant cream for topical use incorporating the composition according to the invention.

The cream comprises five distinct groups of components (phases), named "phase A", "phase B", "phase C", "phase D" and "phase E".

**Table 1**

| *Percentage by weight* | *Component* | *Function* |
|---|---|---|
| | *PHASE A* | |
| 52.100 | Aqua (water) | solvent |
| 0.100 | Disodium EDTA (ethylenediaminetetraacetic acid disodium salt) | chelating agent |
| 5.000 | Pentylene Glycol (1,2-dihydroxy pentane) | humectant |
| 2.000 | Butylene Glycol (1,3-butandiol) | humectant |
| 1.000 | Propylene Glycol (1,2-propanediol) | humectant |
| 0.300 | Hydrogenated Lecithin | hydrogenated phospholipid, active principle |
| 0.300 | Carbomer (carboxyvinyl polymer) | viscosifier |
| 0.100 | Polyglyceril-10 Isostearate (decaglyceryl isostearate) | emulsifier |

| | *PHASE B* | |
|---|---|---|
| 6.000 | Olus Oil (oil of vegetable origin composed mainly of triglycerides of fatty acids) | emollient |
| 4.500 | Prunus Amygdalus Dulcis Oil (oil of seeds of *Prunus amygdalus var. dulcis*) | emollient |
| 4.000 | Glyceryl Stearate | emollient |
| 3.500 | PEG-75 Stearate (polyethylene glycol (75) stearate) | emulsifier |
| 3.000 | Petrolatum | emollient |
| 1.500 | Cetearyl Alcohol (mixture of 1-hexadecanol and 1-octadecanol) | consistency agent |
| 1.000 | PEG-8 Stearate (polyethylene glycol (8) stearate) | emulsifier |
| 1.000 | Isodecyl Laurate | emollient |
| 1.000 | Lauric Acid (dodecanoic acid) | emollient |
| 1.000 | Dimethicone (polydimethylsiloxane) | emollient |
| 1.000 | Glyceryl Laurate | emulsifier |
| 1.000 | Olea Europaea Oil Unsaponifiables (unsaponifiable fraction of oil of *Olea europaea* L.) | emollient |
| 0.500 | Butyrospermum parkii Extract (*Butyrospermum parkii* seed extract) | active principle |
| 0.300 | Stearyl glycyrrhetinate | active principle |
| 0.100 | Paraffin | emollient |

| | *PHASE C* | |
|---|---|---|
| 4.000 | Aqua (water) | solvent |
| 0.200 | Sodium Hydroxide | pH corrector |
| | *PHASE D* | |
| 4.000 | Aqua (water) | solvent |
| 0.500 | Dipotassium Glycyrrhizate | active principle |
| | *PHASE E* | |
| 0.500 | Caprylyl Glycol (1,2-octandiol) | emollient |
| 0.4995 | 1,2-Hexanediol | emollient |
| 0.0005 | Tropolone (2-hydroxycycloheptatrienone) | preservative agent |

The cream having the composition defined in the Table 1 can be prepared according to the procedure disclosed below.

The components of the phase A are mixed in a suitable container (for example, a beaker having appropriate capacity), by heating at 75°C under stirring, until a complete dissolution is obtained. The so obtained solution (phase A) is homogenised for a time equal to 5 minutes.

A second solution is prepared by dissolving at 75°C the components of the phase B; the second solution is added slowly to the phase A, always under stirring. The phase A and the phase B, once mixed, are homogenised for a time equal to 5 minutes.

A third solution is prepared by dissolving at 75°C the components of the phase C; the so obtained third solution (phase C) is added, always at 75°C and under stirring, to the mixture consisting of the phases A and B. The solution containing the three phases A, B, and C is homogenised for a time comprised between 3 and 5 minutes and cooled slowly under stirring to 40°C.

Once the temperature of 40°C has been reached, a fourth solution containing the components of the phase D is added, under stirring, to the solution containing the three phases (A, B, C). The solution containing the four phases A, B, C and D is homogenised for a time equal to 3 minutes, always under stirring.

A fifth solution, containing the components of the phase E, is prepared and added always at 40°C, under stirring, to the solution containing the four phases A, B, C and D. The preparation containing the four phases A, B, C and D is homogenised for a time equal to 3 minutes and cooled under stirring to 20-25°C. Once the aforesaid preparation procedure has been completed, the cream can be packaged - by using known procedures and apparatuses - and subsequently stored.

### Example 2 - Adjuvant cream for topical use

In the Table 2 there is indicated the quali-quantitative composition corresponding to 100 grams of another embodiment of the cream disclosed in Example 1. The cream comprises five distinct groups of components (phases), named "phase A", "phase B", "phase C", "phase D" and "phase E".

**Table 2**

| *Percentage by weight* | *Component* | *Function* |
|---|---|---|
| | *PHASE A* | |
| 53.050 | Aqua (water) | solvent |
| 0.100 | Disodium EDTA (ethylenediaminetetraacetic acid disodium salt) | chelating agent |
| 5.000 | Pentylene Glycol (1,2-dihydroxy pentane) | humectant |
| 2.000 | Butylene Glycol (1,3-butandiol) | humectant |
| 1.000 | Propylene Glycol (1,2-propanediol) | humectant |
| 0.300 | Hydrogenated Lecithin | hydrogenated phospholipid, active principle |

| | *PHASE B* | |
|---|---|---|
| 8.000 | Petrolatum | emollient |
| 4.500 | Prunus Amygdalus Dulcis Oil (oil of seeds of *Prunus amygdalus var. dulcis*) | emollient |
| 3.000 | Glyceryl Stearate | emollient |
| 3.000 | Cetearyl Alcohol (mixture of 1-hexadecanol and 1-octadecanol) | consistency agent |
| 3.000 | PEG-100 Stearate (polyethylene glycol 100 stearate) | emulsifier |
| 3.000 | Dimethicone (polydimethylsiloxane) | emollient |
| 1.000 | Lauric Acid (dodecanoic acid) | emollient |
| 1.000 | Glyceryl Laurate | emulsifier |
| 1.000 | *Butyrospermum parkii* Extract (*Butyrospermum parkii* seed extract) | active principle |
| 1.000 | Stearyl Glycyrrhetinate | active principle |

| | *PHASE C* | |
|---|---|---|
| 4.000 | Aqua (water) | solvent |
| 0.050 | Sodium Hydroxide | pH corrector |

| | *PHASE D* | |
|---|---|---|
| 4.000 | Aqua (water) | solvent |
| 1.000 | Dipotassium Glycyrrhizate | active principle |

| | *PHASE E* | |
|---|---|---|
| 0.500 | Caprylyl Glycol (1,2-octandiol) | emollient |
| 0.4995 | 1,2-Hexanediol | emollient |
| 0.0005 | Tropolone (2-hydroxycycloheptatrienone) | preserving action |

The cream having the composition defined in the Table 2 can be prepared according to the procedure disclosed below.

The components of the phase A are mixed in a suitable container (for example, a beaker having appropriate capacity), by heating at 75°C under stirring, until a complete dissolution is obtained. The so obtained solution (phase A) is homogenised for a time equal to 5 minutes.

A second solution is prepared by dissolving at 75°C the components of the phase B; the second solution is added slowly to the phase A, always under stirring. The phase A and the phase B, once mixed, are homogenised for a time equal to 5 minutes.

A third solution is prepared by dissolving at 75°C the components of the phase C; the so obtained third solution (phase C) is added, always at 75°C and under stirring, to the mixture consisting of the phases A and B. The solution containing the three phases A, B, and C is homogenised for a time comprised between 3 and 5 minutes and cooled slowly to 40°C.

Once the temperature of 40°C has been reached, a fourth solution containing the components of the phase D is added, under stirring, to the solution containing the three phases (A, B, C). The solution containing the four phases A, B, C and D is homogenised for a time equal to 3 minutes under stirring.

A fifth solution, containing the components of the phase E, is added under stirring to the solution containing the four phases A, B, C and D. The solution containing the four phases A, B, C and D is homogenised for a time equal to 3 minutes and cooled under stirring to 20-25°C.

Once the aforesaid preparation procedure has been completed, the cream can be packaged - by using known procedures and apparatuses - and subsequently stored.

### Example 3 - Adjuvant cream for topical use

In the Table 3 there is indicated the quali-quantitative composition corresponding to 100 grams of a further embodiment of the cream disclosed in Example 1. The cream comprises five distinct groups of components (phases), named "phase A", "phase B", "phase C", "phase D" and "phase E".

**Table 3**

| *Percentage by weight* | *Component* | *Function* |
|---|---|---|
| | *PHASE A* | |
| 55.920 | Aqua (water) | solvent |
| 0.100 | Disodium EDTA (ethylenediaminetetraacetic acid disodium salt) | chelating agent |
| 5.000 | Pentylene Glycol (1,2-dihydroxy pentane) | humectant |
| 4.000 | Butylene Glycol (1,3-butandiol) | humectant |
| 0.500 | Hydrogenated Lecithin | hydrogenated phospholipid, active principle |

| | *PHASE B* | |
|---|---|---|
| 5.000 | Petrolatum | emollient |
| 7.000 | Prunus Amygdalus Dulcis Oil (oil of seeds of *Prunus amygdalus var. dulcis*) | emollient |
| 4.000 | Isononyl Isononanoate (3,5,5-trimethylhexanoate of 3,5,5-trimethylhexyl) | emollient |
| 2.000 | Cetearyl Alcohol (mixture of 1-hexadecanol and 1-octadecanol) | consistency agent |
| 3.000 | Polysorbate 60 (Polyoxyethylene (20) sorbitan monostearate) | emulsifier |
| 1.000 | Dimethicone (polydimethylsiloxane) | emollient |
| 1.000 | Lauric Acid (dodecanoic acid) | emollient |
| 1.000 | Glyceryl Laurate | emulsifier |
| 0.250 | *Butyrospermum parkii* Extract (*Butyrospermum parkii* seed extract) | active principle |
| 0.200 | Stearyl Glycyrrhetinate | active principle |

| | *PHASE C* | |
|---|---|---|
| 4.000 | Aqua (water) | solvent |
| 0.030 | Lactic Acid | pH corrector |

| | *PHASE D* | |
|---|---|---|
| 4.000 | Aqua (water) | Solvent |
| 1.000 | Dipotassium Glycyrrhizate | active principle |

| | *PHASE E* | |
|---|---|---|
| 0.500 | Caprylyl Glycol (1,2- octandiol) | emollient |
| 0.4995 | 1,2-Hexanediol | emollient |
| 0.0005 | Tropolone (2-hydroxycycloheptatrienone) | preserving action |

The cream having the composition defined in the Table 3 can be prepared according to the procedure disclosed below.

The components of the phase A are mixed in a suitable container (for example, a beaker having appropriate capacity), by heating at 75°C under stirring, until a complete dissolution is obtained. The so obtained solution (phase A) is homogenised for a time equal to 5 minutes.

A second solution is prepared by dissolving at 75°C the components of the phase B; the second solution is added slowly to the phase A, also under stirring. The phase A and the phase B, once mixed, are homogenised for a time equal to 5 minutes.

A third solution is prepared by dissolving at 75°C the components of the phase C; the so obtained third solution (phase C) is added, always at 75°C and under stirring, to the mixture consisting of the phases A and B. The solution containing the three phases A, B, and C is homogenised for a time comprised between 3 and 5 minutes and cooled slowly to 40°C.

Once the temperature of 40°C has been reached, a fourth solution containing the components of the phase D is added, under stirring, to the solution containing the three phases (A, B, C). The solution containing the four phases A, B, C and D is homogenised for a time equal to 3 minutes under stirring.

A fifth solution, containing the components of the phase E, is prepared and added under stirring to the solution containing the four phases A, B, C and D. The solution containing the four phases A, B, C and D is homogenised for a time equal to 3 minutes and cooled under stirring to 20-25°C.

Once the aforesaid preparation procedure has been completed, the cream can be packaged - by using known procedures and apparatuses - and subsequently stored.

### Example 4 - Adjuvant fluid formulation for topical use

In the Table 4 there is indicated the quali-quantitative composition corresponding to 100 grams of an adjuvant fluid formulation for topical use, incorporating the composition according to the invention. The fluid formulation comprises five distinct groups of components (phases), named "phase A", "phase B", "phase C", "phase D" and "phase E".

**Table 4**

| *Percentage by weight* | *Component* | *Function* |
|---|---|---|
| | *PHASE A* | |
| 58.650 | Aqua (water) | solvent |
| 0.100 | Disodium EDTA (ethylenediaminetetraacetic acid disodium salt) | chelating agent |
| 5.000 | Pentylene Glycol (1,2-dihydroxy pentane) | humectant |
| 1.000 | Hydrogenated Lecithin | hydrogenated phospholipid, active principle |
| 0.150 | Acrylates/C10-30 Alkyl Acrylate Crosspolymer (C10-30 alkyl propenoate, polymer with propenoic acid, butenoic acid and/or alkyl propenoates, product with propenyl sucrose ether or with propenyl 2,2-dihydroxymethyl-1,3-propanediol) | rheological additive |

| | *PHASE B* | |
|---|---|---|
| 6.000 | Olus Oil (oil of vegetable origin mainly consisting of triglycerides of fatty acids) | emollient |
| 4.500 | Prunus Amygdalus Dulcis Oil (oil of seeds of *Prunus amygdalus var. dulcis*) | emollient |
| 1.000 | Glyceryl Stearate | emollient |
| 2.500 | Stearate-21 (stearyl alcohol ether with polyethylene glycol) | emulsifier |
| 2.500 | Steareth-2 (stearyl alcohol ether with polyethylene glycol) | emollient |
| 1.000 | Cetearyl Alcohol (mixture of 1-hexadecanol and 1-octadecanol) | consistency agent |
| 1.000 | Lauric Acid (dodecanoic acid) | emollient |
| 1.000 | Dimethicone (polydimethylsiloxane) | emollient |
| 1.000 | Glyceryl Laurate | emulsifier |
| 1.000 | Olea Europaea Oil Unsaponifiables (unsaponifiable fraction of oil of *Olea europaea* L.) | emollient |
| 1.000 | Butyrospermum parkii Extract (*Butyrospermum parkii* seed extract) | active principle |
| 1.000 | Stearyl Glycyrrhetinate | active principle |

| | *PHASE C* | |
|---|---|---|
| 4.000 | Aqua (water) | solvent |
| 0.100 | Sodium Hydroxide | pH corrector |

| | *PHASE D* | |
|---|---|---|
| 5.000 | Aqua (water) | solvent |
| 1.500 | Dipotassium Glycyrrhizate | active principle |

| | *PHASE E* | |
|---|---|---|
| 0.500 | Caprylyl Glycol (1,2-octandiol) | emollient |
| 0.4995 | 1,2-Hexanediol | emollient |
| 0.0005 | Tropolone (2-hydroxycycloheptatrienone) | preserving action |

The fluid formulation having the composition defined in the Table 4 can be prepared according to the procedure disclosed below. The components of the phase A are mixed in a suitable container (for example, a beaker having appropriate capacity), by heating at 75°C under stirring, until a complete dissolution is obtained. The so obtained solution (phase A) is homogenised for a time equal to 5 minutes.

A second solution is prepared by dissolving at 75°C the components of the phase B; the second solution is added slowly to the phase A, always under stirring. The phase A and the phase B, once mixed, are homogenised for a time equal to 5 minutes.

A third solution is prepared by dissolving the components of the phase C; the so obtained third solution (phase C) is added, always at 75°C and under stirring, to the mixture consisting of the phases A and B. The solution containing the three phases A, B, and C is homogenised for a time comprised between 3 and 5 minutes and cooled slowly to 40°C.

Once the temperature of 40°C has been reached, a fourth solution containing the components of the phase D is added, under stirring, to the solution containing the three phases (A, B, C). The solution containing the four phases A, B, C and D is homogenised for a time equal to 3 minutes under stirring.

A fifth solution, containing the components of the phase E, is prepared and added under stirring to the solution containing the four phases A, B, C and D. The solution containing the four phases A, B, C and D is homogenised for a time equal to 3 minutes and cooled under stirring to 20-25°C.

Once the aforesaid preparation procedure has been completed, the fluid formulation can be packaged - by using known procedures and apparatuses - and subsequently stored.

### Example 5 - Adjuvant gel-cream for topical use

In the Table 5 there is indicated the quali-quantitative composition corresponding to 100 grams of an adjuvant gel-cream for topical use, incorporating the composition according to the invention.

The gel-cream comprises five distinct groups of components (phases), named "phase A", "phase B", "phase C", "phase D" and "phase E".

**Table 5**

| *Percentage by weight* | *Component* | *Function* |
|---|---|---|
| | *PHASE A* | |
| 74.900 | Aqua (water) | solvent |
| 0.100 | Disodium EDTA (ethylenediaminetetraacetic acid disodium salt) | chelating agent |
| 5.000 | Pentylene Glycol (1,2-dihydroxy pentane) | humectant |
| 1.500 | Hydrogenated Lecithin | hydrogenated phospholipid, active principle |

| | *PHASE B* | |
|---|---|---|
| 3.000 | Olus Oil (oil of vegetable origin mainly consisting of triglycerides of fatty acids) | emollient |
| 2.000 | Prunus Amygdalus Dulcis Oil (oil of seeds of *Prunus amygdalus var. dulcis*) | emollient |
| 1.500 | Butyrospermum parkii Extract (*Butyrospermum parkii* seed extract) | active principle |
| 1.500 | Stearyl Glycyrrhetinate | active principle |

| | *PHASE C* | |
|---|---|---|
| 3.000 | Hydroxyethil Acrylate Sodium/ Acryloyldimethil Taurate Copolymer, Squalane, (2,6,10,15,19,23-hexamethyl tetracosane) Polysorbate 60 (Polyoxyethylene (20) sorbitan monostearate) | emulsifier/ gelling agent |

| | *PHASE D* | |
|---|---|---|
| 5.000 | Aqua (water) | solvent |
| 1.500 | Dipotassium Glycyrrhizate | active principle |

| | *PHASE E* | |
|---|---|---|
| 0.500 | Caprylyl Glycol (1,2-octandiol) | emollient |
| 0.4995 | 1,2-Hexanediol | emollient |
| 0.0005 | Tropolone (2-hydroxycycloheptatrienone) | preserving action |

The gel-cream having the composition defined in the Table 5 can be prepared according to the procedure disclosed below.

The components of the phase A are mixed in a suitable container (for example, a beaker having appropriate capacity), by heating at 75°C under stirring, until a complete dissolution is obtained. The so obtained solution (phase A) is homogenised for a time equal to 5 minutes.

A second solution is prepared by dissolving at 75°C the components of the phase B; the second solution is added slowly to the phase A, always under stirring. The phase A and the phase B, once mixed, are homogenised for a time equal to 5 minutes.

A third solution is prepared by dissolving the components of the phase C; the so obtained third solution (phase C) is added, always at 75°C and under stirring, to the mixture consisting of the phases A and B. The solution containing the three phases A, B, and C is homogenised for a time comprised between 3 and 5 minutes and cooled slowly under stirring to 40°C.

Once the temperature of 40°C has been reached, a fourth solution containing the components of the phase D is added, under stirring, to the solution containing the three phases (A, B, C). The solution containing the four phases A, B, C and D is homogenised for a time equal to 3 minutes under stirring.

A fifth solution, containing the components of the phase E, is prepared and added under stirring to the solution containing the four phases A, B, C and D. The solution containing the four phases A, B, C and D is homogenised for a time equal to 3 minutes and cooled under stirring to 20-25°C.

Once the aforesaid preparation procedure has been completed, the gel-cream can be packaged - by using known procedures and apparatuses - and subsequently stored.

### Example 6 - Adjuvant serum-gel for topical use

In the Table 6 there is indicated the quali-quantitative composition corresponding to 100 grams of an adjuvant serum-gel for topical use, incorporating the composition according to the invention.

The serum-gel comprises five distinct groups of components (phases), named "phase A", "phase B", "phase C", "phase D" and "phase E".

**Table 6**

| *Percentage by weight* | *Component* | *Function* |
|---|---|---|
| | *PHASE A* | |
| 78.900 | Aqua (water) | Solvent |
| 0.100 | Disodium EDTA (ethylenediaminetetraacetic acid disodium salt) | chelating agent |
| 5.000 | Pentylene Glycol (1,2-dihydroxy pentane) | humectant |
| 0.500 | Hydrogenated Lecithin | hydrogenated phospholipid, active principle |

| | *PHASE B* | |
|---|---|---|
| 3.000 | Olus Oil (oil of vegetable origin mainly consisting of triglycerides of fatty acids) | emollient |
| 2.000 | Prunus Amygdalus Dulcis Oil (oil of seeds of *Prunus amygdalus var. dulcis*) | emollient |
| 0.500 | Butyrospermum parkii Extract (*Butyrospermum parkii* seed extract) | active principle |
| 0.500 | Stearyl Glycyrrhetinate | active principle |

| | *PHASE C* | |
|---|---|---|
| 3.000 | Hydroxyethil Acrylate Sodium/ Acryloyldimethil Taurate Copolymer, Squalane, | emulsifier/ gelling agent |
| | (2,6,10,15,19,23-hexamethyl tetracosane) Polysorbate 60 (Polyoxyethylene (20) sorbitan monostearate) | |

| | *PHASE D* | |
|---|---|---|
| 5.000 | Aqua (water) | solvent |
| 0.500 | Dipotassium Glycyrrhizate | active principle |

| | PHASE E | |
|---|---|---|
| 0.500 | Caprylyl Glycol (1,2-octandiol) | emollient |
| 0.4995 | 1,2-Hexanediol | emollient |
| 0.0005 | Tropolone (2-hydroxycycloheptatrienone) | preserving action |

The serum-gel having the composition defined in the Table 6 can be prepared according to the procedure disclosed below.

The components of the phase A are mixed in a suitable container (for example, a beaker having appropriate capacity), by heating at 75°C under stirring, until a complete dissolution is obtained. The so obtained solution (phase A) is homogenised for a time equal to 5 minutes.

A second solution is prepared by dissolving at 75°C the components of the phase B; the second solution is added slowly to the phase A, always under stirring. The phase A and the phase B, once mixed, are homogenised for a time equal to 5 minutes.

A third solution is prepared by dissolving the components of the phase C; the so obtained third solution (phase C) is added, always at 75°C and under stirring, to the mixture consisting of the phases A and B. The solution containing the three phases A, B, and C is homogenised for a time comprised between 3 and 5 minutes and cooled slowly to 40°C.

Once the temperature of 40°C has been reached, a fourth solution containing the components of the phase D is added, under stirring, to the solution containing the three phases (A, B, C). The solution containing the four phases A, B, C and D is homogenised for a time equal to 3 minutes and cooled under stirring.

A fifth solution, containing the components of the phase E, is prepared and added under stirring to the solution containing the four phases A, B, C and D. The solution containing the four phases A, B, C and D is homogenised for a time equal to 3 minutes and cooled under stirring to 20-25°C.

Once the aforesaid preparation procedure has been completed, the serum-gel can be packaged - by using known procedures and apparatuses - and subsequently stored.

The results of the clinical experimentation (conducted by Professor Aurora Parodi of the Department of Health Sciences, Dermatology Section, University of Genoa) are disclosed briefly below, which clinical experimentation has enabled the anti-inflammatory effectiveness (in particular, the capacity to reduce an erythema caused by exposure to ultraviolet radiation) of the composition according to the invention to be verified. During the experimentation, the anti-inflammatory activity of the composition according to the invention has been compared with the anti-inflammatory activity of a known corticosteroid.

The composition according to the invention was used in the form of a cream and hydrocortisone butyrate has been used as a corticosteroid.

A sample consisting of 8 healthy volunteers, 6 of which were of female sex and 2 were of male sex, has been tested.

The average age of the subjects was 33.3, with an age range comprised between 24 and 70. Two subjects were of phototype II, 5 subjects were of phototype III, 1 subject was of phototype I. Each subject has been irradiated by using 6 broadband (type TL12) UVB (short UV) lamps, having an emission of 0.6 mW/cm².

For each subject, the MED UVB has been determined, i.e. the Minimal Erythema Dose induced by the UVB radiation. The "Minimal Erythema Dose" is defined as the minimum irradiation dose that is able to induce an erythema that is perceptible and within clear limits in the irradiated skin area.

At an initial time T0, the skin of the volar part of the forearm of each subject has been irradiated with a UVB dose equal to 2MED, for a time varying between 4 minutes 30 seconds and 6 minutes, in 5 distinct areas. The irradiated area had not been exposed to sunlight for at least 30 days.

In the volar part of the forearm of each subject, proceeding from the elbow to the wrist, the 5 irradiated areas have been defined and treated in the following manner:
- Area 1, or control area, which has been irradiated only but not treated, i.e. neither the composition according to the invention or the hydrocortisone butyrate have been applied;
- Area 2, which has been irradiated and treated with the composition according to the invention immediately after the irradiation and then twice a day for 6 days;
- Area 3, which has been irradiated and treated with hydrocortisone butyrate immediately after the irradiation and then twice a day for 6 days;
- Area 4, which has been irradiated and treated with the composition according to the invention 4 hours after the irradiation and then twice a day for 6 days;
- Area 5, which has been irradiated and treated with hydrocortisone butyrate 4 hours after the irradiation and then twice a day for 6 days.

The colour of the skin in the five aforesaid areas has been subsequently read using a reflectance colorimeter of known type (Chroma Meter, Minolta) . The colour has been read after 24 hours (time T1), after 48 hours (time T2) and after 6 days (time T3). For the reading, a colour classification and chromatic analysis system of known type has been used, i.e. the CIE system based on the L*a*b* coordinates. The colorimetric values relating to the coordinate a* - which enables the intensity of the redness and thus the erythema to be determined - are considered as difference with the data obtained from the normal skin of the volar part of the forearm (non-irradiated and non-treated area) and are compared with the control (irradiated and non-treated area). The colorimetric values have been calculated and examined by using a statistical method of known type, i.e. the Student's T test for paired data.

The readings made on the 8 subjects have given the results, expressed as averages ± standard deviation, set out in the following Table B:

**Table B**

| | time T1 | time T2 | time T3 |
|---|---|---|---|
| Area 1 | 11 ± 2.6 | 10.62 ± 3.49 | 5.38 ± 2.8 |
| Area 2 | 9.72 ± 2.26 | 9.88 ± 3.34* | 5.2 ± 2.44 |
| | | *p = 0.05* | |
| Area 3 | 9.15 ± 2.56** | 9.64 ± 3.11** | 5.24 ± 2.54 |
| | *p = 0.006* | *p = 0.04* | |
| Area 4 | 8.49 ± 2.04** | 9.42 ± 3.01** | 5.32 ± 2.93 |
| | *p = 0.001* | *p = 0.03* | |
| Area 5 | 8.31 ± 2.47** | 8.52 ± 3.06** | 4.81 ± 3.16 |
| | *p = 0.002* | *p = 0.0009* | |

In the Table B, the values marked by the symbol "*" (single asterisk) indicate results that are statistically significant compared with the control, whilst the values marked by the symbol "**" (double asterisk) indicate results that are highly significant, on a statistical point of view, compared with the control.

The experimental results obtained indicate that at the time T1 (24 hours after the irradiation), the anti-inflammatory efficacy (reduction of the erythema) of the composition according to the invention is greater in the areas in which the treatment is made 4 hours after the irradiation (Area 4), compared with the areas in which the treatment is made immediately after the irradiation (Area 2). The aforesaid results (8.49 ± 2.04) are nevertheless not statistically different compared with those obtained by applying hydrocortisone butyrate (8.31 ± 2.47).

At the time T2 (48 hours after the irradiation), the composition according to the invention reduces the erythema in a statistically significant manner compared with the control and in a slightly less (9.42 ± 3.01), but statistically not significant, manner compared with the hydrocortisone butyrate (8.52 ± 3.06).

At the time T3 (6 days after the irradiation), the erythema has almost completely disappeared on all the treated areas and, in particular, the erythema has disappeared in uniform manner both in the areas treated with the composition according to the invention and in the areas treated with hydrocortisone butyrate. In the light of the experimental results disclosed above, it can be stated that the composition according to the invention is able to perform an anti-inflammatory activity that is substantially comparable with that performed by corticosteroids - without, however, inducing the undesired side effects caused by these drugs - and is therefore effectively usable as a soothing adjuvant composition in the topical treatment of skin disreactivity.

## Claims

1. Composition for topical use, comprising a hydrosoluble salt of the glycyrrhizic acid, a liposoluble ester of the 18-β-glycyrrhetic acid and a *Butyrospermum parkii* seed extract, wherein:
- said hydrosoluble salt of the glycyrrhizic acid is selected from a group consisting of dipotassium glycyrrhizate, disodium glycyrrhizate and ammonium glycyrrhizate;
- in said liposoluble ester, said 18-β-glycyrrhetic acid is esterified with a compound that is selected from a group consisting of stearic acid, glyceric acid, succinic acid and pyridoxine;
- said *Butyrospermum parkii* seed extract is enriched in triterpene esters.

2. Composition according to claim 1, further comprising hydrogenated lecithin.

3. Composition according to any preceding claim, wherein said hydrosoluble salt of the glycyrrhizic acid is contained in a percentage in weight comprised between 0.1% and 10%.

4. Composition according to claim 3, wherein said hydrosoluble salt of the glycyrrhizic acid is contained in a percentage by weight comprised between 0.5% and 2%.

5. Composition according to any preceding claim, wherein said liposoluble ester of the 18-β-glycyrrhetic acid is contained in a percentage by weight comprised between 0.1% and 10%.

6. Composition according to claim 5, wherein said liposoluble ester of the 18-β-glycyrrhetic acid is contained in a percentage by weight comprised between 0.2% and 2%.

7. Composition according to any preceding claim, wherein said *Butyrospermum parkii* seed extract is contained in a percentage by weight comprised between 0.1% and 10%.

8. Composition according to claim 7, wherein said *Butyrospermum parkii* seed extract is contained in a percentage by weight comprised between 0.25% and 1.5%.

9. Composition according to claim 2, or according to any one of claims 3 to 8 as appended to claim 2, wherein said hydrogenated lecithin is contained in a percentage by weight comprised between 0.1% and 10%.

10. Composition according to claim 9, wherein said hydrogenated lecithin is contained in a percentage by weight comprised between 0.3% and 2%.

11. Cosmetic adjuvant composition, comprising the composition according to any preceding claim.

12. Composition according to any preceding claim and made in a form that is selected from a group consisting of: cream, fluid, emulsion, gel, gel-cream, serum-gel, paste, mousse, spray.

13. Composition according to any preceding claim for use in the treatment of a skin disreactivity, wherein said skin disreactivity is an abnormal, inflammatory or immune, response of the skin tissues to an exogenous or endogenous stimulus.

14. Composition for use according to claim 13, wherein said skin disreactivity is selected from a group consisting of: contact dermatitises, allergic dermatitises, irritative dermatitises, atopic dermatitises, seborrhoeic dermatitises, solar erythemas, radiodermatitises, skin stresses.

## Patentansprüche

1. Zusammensetzung zur topischen Verwendung, welche ein wasserlösliches Salz der Glycyrrhizinsäure, einen fettlöslichen Ester der 18-β-Glycyrrhetinsäure und einen *Butyrospermum parkii* Samenextrakt aufweist, wobei:
- das wasserlösliche Salz der Glycyrrhizinsäure ausgewählt ist aus der Gruppe bestehend aus Dikalium-Glycyrrhizinat, Dinatrium-Glycyrrhizinat und Ammonium-Glycyrrhizinat;
- die 18-β-Glycyrrhetinsäure des fettlöslichen Ester mit einer Verbindung verestert ist, die ausgewählt ist aus der Gruppe bestehend aus Stearinsäure, Glyzerinsäure, Bernsteinsäure und Pyridoxin;
- der *Butyrospermum parkii* Samenextrakt in Triterpen-Estern angereichert ist.

2. Zusammensetzung nach Anspruch 1, die ferner ein hydriertes Lecithin aufweist.

3. Zusammensetzung nach einem der vorstehenden Ansprüche, wobei das wasserlösliche Salz der Glycyrrhizinsäure mit zwischen 0,1% und 10% Gewichtsprozent enthalten ist.

4. Zusammensetzung nach Anspruch 3, wobei das wasserlösliche Salz der Glycyrrhizinsäure mit zwischen 0,5% und 2% Gewichtsprozent enthalten ist.

5. Zusammensetzung nach einem der vorstehenden Ansprüche, wobei der fettlösliche Ester der 18-β-Glycyrrhetinsäure mit zwischen 0,1% und 10% Gewichtsprozent enthalten ist.

6. Zusammensetzung nach Anspruch 5, wobei der fettlösliche Ester der 18-β-Glycyrrhetinsäure mit zwischen 0,2% und 2% Gewichtsprozent enthalten ist.

7. Zusammensetzung nach einem der vorstehenden Ansprüche, wobei der *Butyrospermum parkii* Samenextrakt mit zwischen 0,1% und 10% Gewichtsprozent enthalten ist.

8. Zusammensetzung nach Anspruch 7, wobei der *Butyrospermum parkii* Samenextrakt mit zwischen 0,25% und 1,5% Gewichtsprozent enthalten ist.

9. Zusammensetzung nach Anspruch 2 oder nach einem der Ansprüche 3 bis 8, sofern von Anspruch 2 abhängig, wobei das hydrierte Lecithin mit zwischen 0,1% und 10% Gewichtsprozent enthalten ist.

10. Zusammensetzung nach Anspruch 9, wobei das hydrierte Lecithin mit zwischen 0,3% und 2% Gewichtsprozent enthalten ist.

11. Kosmetische Adjuvans-Zusammensetzung, aufweisend die Zusammensetzung nach einem der vorstehenden Ansprüche.

12. Zusammensetzung nach einem der vorstehenden Ansprüche und hergestellt in einer Form, die ausgewählt ist aus der Gruppe bestehend aus: Creme, Fluid, Emulsion, Gel, Gelcreme, Serumgel, Paste, Mousse, Spray.

13. Zusammensetzung nach einem der vorstehenden Ansprüche zur Verwendung bei der Behandlung einer Hautirritation, wobei die Hautirritation eine abnormale, entzündliche oder immune Antwort der Hautgewebe auf einen exogenen oder endogenen Reiz ist.

14. Zusammensetzung zur Verwendung nach Anspruch 13, wobei die Hautirritation ausgewählt ist aus der Gruppe bestehend aus: Kontaktdermatitis, allergische Dermatitis, irritative Dermatitis, atopische Dermatitis, seborrhoische Dermatitis, Sonnenerythema, Radiodermatitis, Hautstress.

## Revendications

1. Composition pour une utilisation topique, comprenant un sel hydrosoluble de l'acide glycyrrhizique, un ester liposoluble de l'acide 18-β-glycyrrhétique et un extrait de graines de *Butyrospermum parkii*, dans laquelle :
- ledit sel hydrosoluble de l'acide glycyrrhizique est choisi dans un groupe constitué de glycyrrhizate dipotassique, glycyrrhizate disodique et glycyrrhizate d'ammonium ;
- dans ledit ester liposoluble, ledit acide 18-β-glycyrrhétique est estérifié avec un composé qui est choisi dans un groupe constitué d'acide stéarique, acide glycérique, acide succinique et pyridoxine ;
- ledit extrait de graines de *Butyrospermum parkii* est enrichi en esters de triterpène.

2. Composition selon la revendication 1, comprenant en outre de la lécithine hydrogénée.

3. Composition selon l'une quelconque des revendications précédentes, dans laquelle ledit sel hydrosoluble de l'acide glycyrrhizique est contenu dans un pourcentage en poids compris entre 0,1 % et 10 %.

4. Composition selon la revendication 3, dans laquelle ledit sel hydrosoluble de l'acide glycyrrhizique est contenu dans un pourcentage en poids compris entre 0,5 % et 2 %.

5. Composition selon l'une quelconque des revendications précédentes, dans laquelle ledit ester liposoluble de l'acide 18-β-glycyrrhétique est contenu dans un pourcentage en poids compris entre 0,1 % et 10 %.

6. Composition selon la revendication 5, dans laquelle ledit ester liposoluble de l'acide 18-β-glycyrrhétique est contenu dans un pourcentage en poids compris entre 0,2 % et 2 %.

7. Composition selon l'une quelconque des revendications précédentes, dans laquelle ledit extrait de graines de *Butyrospermum parkii* est contenu dans un pourcentage en poids compris entre 0,1 % et 10 %.

8. Composition selon la revendication 7, dans laquelle ledit extrait de graines de *Butyrospermum parkii* est contenu dans un pourcentage en poids compris entre 0,25 % et 1,5 %.

9. Composition selon la revendication 2, ou selon l'une quelconque des revendications 3 à 8 telles qu'annexées à la revendication 2, dans laquelle ladite lécithine hydrogénée est contenue dans un pourcentage en poids compris entre 0,1 % et 10 %.

10. Composition selon la revendication 9, dans laquelle ladite lécithine hydrogénée est contenue dans un pourcentage en poids compris entre 0,3 % et 2 %.

11. Composition d'adjuvant cosmétique, comprenant la composition selon l'une quelconque des revendications précédentes.

12. Composition selon l'une quelconque des revendications précédentes et fabriquée sous une forme qui est choisie dans un groupe constitué de : crème, liquide, émulsion, gel, gel-crème, sérum-gel, pâte, mousse, pulvérisation.

13. Composition selon l'une quelconque des revendications précédentes pour une utilisation dans le traitement d'une dysréactivité cutanée, où ladite dysréactivité cutanée est une réponse anormale, inflammatoire ou immunitaire des tissus cutanés à un stimulus exogène ou endogène.

14. Composition pour une utilisation selon la revendication 13, où ladite dysréactivité cutanée est choisie dans un groupe constitué de : dermatites de contact, dermatites allergiques, dermatites irritatives, dermatites atopiques, dermatites séborrhéiques, érythèmes solaires, radio-dermatites, stress cutanés.
